# FASCICULE DE BREVET EUROPEEN

(11) **EP 3 297 578 B1**
(45) Date de publication et mention de la délivrance du brevet: **24.04.2019**
(21) Numéro de dépôt: 16727770.6
(22) Date de dépôt: 17.05.2016
(51) Int. Cl.: A61F 2/54, A61F 2/60, A61F 2/64, A61F 2/66

(54) **PROCEDE DE COMMANDE D'UNE PROTHESE CONTROLEE PAR UN MICROCONTRÔLEUR, ET PROTHESE EQUIPEE D'UN MICROCONTRÔLEUR POUR LA MISE EN UVRE DU PROCEDE DE COMMANDE**
METHODE ZUR STEUERUNG EINER PROTHESE MIT EINEM MIKROPROZESSOR UND PROTHESE MIT EINEM MIKROPROZESSOR ZUR AUSFÜHRUNG DIESER METHODE
CONTROL PROCEDURE FOR A PROSTHESIS CONTROLLED BY A MICROCONTROLLER AND PROSTHESIS COMPRISING A MICROCONTROLLER FOR EXECUTING THIS CONTROL PROCEDURE

(30) Priorité: 19.05.2015 FR 1554477
(43) Date de publication de la demande: 28.03.2018
(73) Titulaire: Proteor, 21850 Saint-Apollinaire (FR)
(72) Inventeur: BONNET, Xavier, 21000 Dijon (FR); DJIAN, Francis, 21110 Genlis (FR)
(74) Mandataire: Cabinet Laurent & Charras
(86) Numéro de dépôt international: PCT/FR2016/051161
(87) Numéro de publication internationale: WO 2016/185132

(56) Documents cités:
- EP-A2- 2 842 522
- US-A1- 2011 264 238
- US-A1- 2013 261 766

## Description

### DOMAINE TECHNIQUE

La présente invention concerne un procédé de commande d'une prothèse comprenant une ou plusieurs articulation(s) contrôlée(s) par un microcontrôleur, telle qu'une prothèse de genou, de cheville, ou de genou-cheville destinée à des personnes amputées du membre inférieur par exemple. L'invention concerne également une prothèse équipée d'un microcontrôleur pour la mise en oeuvre dudit procédé de commande.

### ART ANTERIEUR

Il est connu de l'état de la technique des prothèses comprenant une articulation contrôlée par un actionneur dont le degré de freinage en flexion est piloté par un microcontrôleur à partir de données par exemple issues d'un gyroscope et d'un accéléromètre aptes à mesurer respectivement la vitesse angulaire et l'accélération d'au moins une partie de la prothèse.

Un tel type de prothèse connu, telle que par exemple une prothèse fémorale genou-cheville notamment décrite dans la demande internationale de brevet n° WO 2014/016424, au nom du Demandeur, comprend un système actionneur hydraulique agencé pour contrôler l'articulation du genou et de la cheville et dont le degré de freinage en flexion est piloté par au moins une vanne, elle-même pilotée par un moteur. Le moteur est quant à lui piloté par le microcontrôleur à partir de données issues par exemple de capteurs, tels qu'un gyroscope détectant la vitesse angulaire du tibia par rapport à l'axe médio-latéral, un accéléromètre mesurant l'accélération du tibia selon l'axe antéro-postérieur, un capteur indiquant l'angle du genou, et une bielle instrumentée par une jauge de déformation permettant d'évaluer le moment de la cheville par rapport à un axe médio-latéral.

Le traitement des données issues des capteurs permet la détection des différentes phases de la marche et l'adaptation du genou et de la cheville aux différentes situations parmi la marche à plat, en pente, en escalier, les transitions assis-debout, etc...

Cependant, l'autonomie énergétique de ce type de prothèse, qui est un facteur primordial pour l'utilisateur, dépend de la capacité de la batterie embarquée, de la consommation en tant que telle, et peut être largement augmentée.

Le document EP 2 842 522 décrit une prothèse et un procédé de commande selon l'art antérieur

### EXPOSE DE L'INVENTION

L'un des buts de l'invention est donc de remédier à ces inconvénients en proposant un procédé de commande d'une prothèse permettant d'augmenter l'autonomie de fonctionnement de ladite prothèse.

A cet effet, il est proposé un procédé de commande d'une prothèse comprenant au moins une articulation contrôlée par un actionneur piloté par un microcontrôleur à partir de données issues d'un gyroscope et d'un accéléromètre aptes à mesurer respectivement la vitesse angulaire et l'accélération d'au moins une partie de la prothèse.

Conformément à l'invention, le procédé consiste en ce que, pendant une période de temps déterminée, le microcontrôleur surveille la mesure donnée par le gyroscope de sorte que ledit microcontrôleur passe dans un état de veille en réduisant l'alimentation électrique d'au moins un organe électronique de la prothèse, et de préférence de tous les organes électroniques, à l'exception de celle de l'accéléromètre, lorsque ledit microcontrôleur détecte que la valeur absolue de la mesure donnée par le gyroscope est inférieure à un seuil donné pendant la période de temps déterminée.

Ainsi, lorsque le gyroscope de la prothèse est stable, c'est-à-dire qu'il évalue une absence de mouvement de la prothèse, le microcontrôleur passe à l'état de veille et les organes électroniques de la prothèse sont mis en veille. La mise en veille permet ainsi de réduire la consommation électrique en réduisant l'alimentation des organes électroniques lorsqu'ils ne sont pas utilisés.

Avantageusement, pendant la période de temps déterminée durant laquelle le microcontrôleur surveille la mesure donnée par le gyroscope, l'accéléromètre calcule et enregistre la moyenne de l'accélération, de sorte que lorsque l'accéléromètre détecte un écart déterminé entre la valeur de l'accélération mesurée pendant l'état de veille et la valeur de la moyenne de l'accélération enregistrée avant le passage à l'état de veille du microcontrôleur, l'accéléromètre génère un signal apte à faire passer le microcontrôleur à nouveau dans un état de fonctionnement normal en rétablissant l'alimentation électrique normale du ou des organes électroniques de la prothèse.

De cette manière, le procédé permet de passer automatiquement le microcontrôleur de l'état veille à l'état de fonctionnement normal uniquement grâce à l'accéléromètre qui a une consommation d'énergie relativement faible. Le procédé permet donc de réduire ou de couper l'alimentation uniquement lorsque la prothèse ne nécessite aucune alimentation électrique et de passer à nouveau à l'état de fonctionnement normal au moindre besoin en alimentation électrique. L'alimentation de la prothèse est donc gérée en temps réel, et sur mesure en fonction du besoin réel en électricité des organes électroniques. La consommation électrique est optimisée et l'autonomie est augmentée en conséquence.

Ainsi, la sortie de l'état de veille se fait par la détection d'une variation de la moyenne de l'accélération mesurée par rapport à la moyenne de l'accélération avant l'entrée en veille. De cette manière, la prothèse ne se réactive pas de manière inopinée lorsqu'elle reçoit un léger choc. La sortie de l'état de veille est calculée de manière relative par rapport à l'environnement et la situation de la prothèse avant la veille.

L'accéléromètre est capable de calculer et d'enregistrer lui-même les informations relatives aux moyennes de l'accélération car il embarque un calculateur. Le calculateur embarqué dans l'accéléromètre est capable de faire, de manière autonome, le calcul des moyennes et de la variation entre les moyennes. L'accéléromètre consomme très peu d'énergie, même avec le calculateur spécifique intégré. Pendant l'état de veille, le calculateur principal de la prothèse est également coupé car l'accéléromètre inclut des moyens suffisants de calcul.

Selon une forme de réalisation particulière du procédé de contrôle selon l'invention, le gyroscope est agencé de sorte à mesurer la vitesse angulaire d'un segment lié à l'articulation prothétique par rapport à un axe médio-latéral, et l'accéléromètre est agencé de sorte à mesurer l'accélération d'un segment lié à l'articulation prothétique selon un axe antéro-postérieur.

Dans cette configuration, le microcontrôleur contrôle, via différents actionneurs, les propriétés physiques d'une ou de plusieurs articulations de la prothèse lors de la déambulation dans différentes situations que ce soit lors de la marche ou le franchissement de pentes ou d'escaliers. Ce contrôle des articulations peut être réalisé à partir d'une machine d'états. Cette machine d'états contient différents états liés aux phases ou aux instants du cycle de marche (premier double appui, milieu d'appui, fin d'appui unipodal, phase de pré-oscillation, milieu de phase oscillante, fin de phase oscillante, etc...). Le contrôle des articulations de la prothèse est réalisé via des moteurs pour contrôler une propriété de la prothèse dans chacun de ces états (amortissement, position d'une butée hydraulique, etc...). Différents capteurs peuvent être utilisés pour définir les transitions entre ces différents états comme par exemple un capteur indiquant l'angle du genou ou des moyens permettant d'évaluer le moment de la cheville par rapport à un axe médio-latéral, telle qu'une pièce instrumentée par une jauge de déformation par exemple.

Ainsi, le microcontrôleur passe à l'état de veille lorsqu'il détecte, d'une part, que la mesure donnée par le gyroscope est inférieure à un seuil donné pendant une période de temps minimum, et d'autre part, que la prothèse est dans un état permettant d'assurer la sécurité de l'utilisateur pendant l'état de veille, par exemple un état dans lequel la résistance de l'articulation est élevée, c'est-à-dire supérieure à un seuil déterminé, ou dans un état dans lequel l'articulation est bloquée.

Ainsi, si la machine d'états contient différents états, le passage dans un état de veille n'est possible qu'à partir des états où la prothèse assure la sécurité de l'utilisateur.

En d'autres termes, dans cette configuration, lorsque ledit microcontrôleur détecte que la mesure donnée par le gyroscope est inférieure à un seuil donné pendant une période de temps minimum, cela signifie que le segment prothétique auquel le gyroscope est lié n'a pas de mouvements de rotation. Ainsi, soit le segment prothétique est en translation pure, soit il est immobile. Dans ces deux cas, il n'est plus utile de vouloir détecter de transition entre les états de la machine d'états, ni de vouloir modifier le comportement des articulations prothétiques. Il est alors possible de passer une partie du système dans un état de veille pour économiser de l'énergie et augmenter l'autonomie de fonctionnement de la prothèse.

De préférence, lorsque le microcontrôleur passe à l'état de veille, celui-ci coupe l'alimentation électrique d'au moins un organe électronique, et de préférence de tous les organes électroniques de la prothèse, à l'exception de celle de l'accéléromètre.

De préférence, le signal généré par l'accéléromètre permet au microcontrôleur de passer à nouveau à l'état de fonctionnement normal et le système retourne dans l'état de la machine d'états dans lequel était le système avant son passage à l'état de veille.

L'invention concerne également une prothèse, telle qu'une prothèse de genou, de cheville, ou de genou-cheville, comprenant au moins une articulation contrôlée par un actionneur piloté par un microcontrôleur à partir de données issues d'un gyroscope et d'un accéléromètre aptes à mesurer respectivement la vitesse angulaire et l'accélération d'au moins une partie de la prothèse.

Selon l'invention, la prothèse comprend un microcontrôleur apte à surveiller, pendant une période de temps déterminée, la mesure donnée par le gyroscope et à passer dans un état de veille en réduisant ou en coupant l'alimentation électrique d'au moins un organe électronique de la prothèse, à l'exception de celle de l'accéléromètre, lorsque ledit microcontrôleur détecte que la valeur absolue de la mesure donnée par le gyroscope est inférieure à un seuil donné pendant la période de temps déterminée.

### BREVE DESCRIPTION DES FIGURES

D'autres avantages et caractéristiques ressortiront mieux de la description qui va suivre, donnée à titre d'exemple non limitatif, d'un procédé de commande d'une prothèse de genou, de cheville, ou de genou-cheville conforme à l'invention, en référence aux figures annexées dans lesquelles :
- la figure 1 illustre une prothèse de genou-cheville adaptée pour la mise en oeuvre du procédé de commande ;
- la figure 2 illustrant un extrait d'une machine d'états sur laquelle est basé un logiciel embarqué dans le microcontrôleur.

### DESCRIPTION DETAILLEE DE L'INVENTION

L'invention concerne un procédé de commande d'une prothèse pour la mise en veille de ses organes électroniques dans le but de diminuer la consommation d'énergie de ladite prothèse et d'augmenter son autonomie de fonctionnement.

Le procédé selon l'invention est applicable à tout type de prothèse ou orthèse intégrant des organes électroniques, telle que par exemple une prothèse de genou, de cheville, ou de genou-cheville destinée à des personnes amputées du membre inférieur.

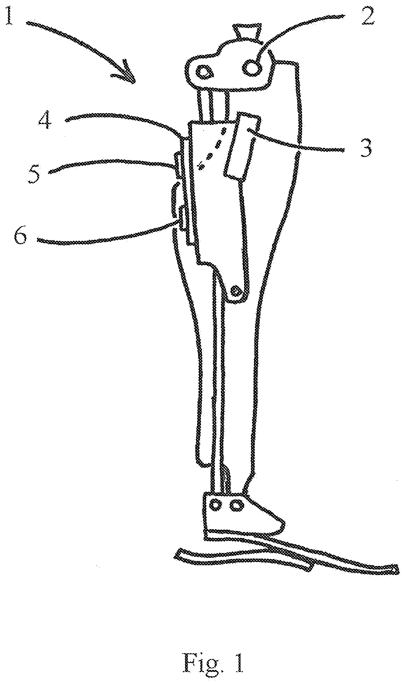

En référence à la figure 1, le procédé selon l'invention sera décrit ci-après en relation avec une prothèse (1) fémorale genou-cheville comprenant un segment fémoral apte à être fixé à une connexion fémorale, et un segment tibial articulé, d'une part, au segment fémoral autour d'une articulation (2) reproduisant les mouvements du genou et, d'autre part, à un pied autour d'une articulation reproduisant les mouvements de la cheville.

La prothèse (1) comprend par exemple un système actionneur (3) hydraulique agencé pour contrôler l'articulation (2) du genou et de la cheville et dont le degré de freinage en flexion est piloté par une électrovanne, elle-même pilotée par un moteur.

Le système actionneur (3) hydraulique, non représenté en détail, comporte par exemple un premier vérin hydraulique double effet dont les extrémités sont respectivement solidaires du segment fémoral et du segment tibial, et un second vérin hydraulique double effet dont les extrémités sont respectivement solidaires du segment tibial et du pied par l'intermédiaire d'une bielle.

Le moteur est, quant à lui, piloté par un microcontrôleur (4) apte à déterminer la phase de marche, telle que la phase d'appui ou la phase oscillante, et la situation telle que la descente d'un escalier, une pente ou une station debout ou analogue à partir de données issues de capteurs placés sur l'articulation (2) de genou et/ou l'articulation de cheville et/ou le tibia et/ou les vérins.

Ces capteurs sont par exemple un gyroscope (5) détectant la vitesse angulaire du tibia par rapport à l'axe médio-latéral, un accéléromètre (6) mesurant l'accélération du tibia selon l'axe antéro-postérieur, un capteur indiquant l'angle du genou, et une bielle instrumentée par une jauge de déformation permettant d'évaluer le moment de la cheville par rapport à un axe médio-latéral.

Ledit microcontrôleur (4) pilote la ou les électrovannes de manière à faire varier les résistances à la flexion ou l'extension de l'articulation (2) de genou et/ou de l'articulation de cheville. La flexion du genou autorise par exemple une dorsiflexion de cheville proportionnelle au mouvement du genou au cours de la phase d'appui et la flexion du genou entraine une dorsiflexion de la cheville au cours de la phase oscillante.

En fonction de la phase de la marche et de la situation déterminées par le microcontrôleur (4), le procédé selon l'invention permet de passer ledit microcontrôleur (4) dans un état de veille en réduisant ou en coupant l'alimentation électrique d'au moins un organe électronique de la prothèse (1), et de préférence de tous les organes électroniques, à l'exception de celle de l'accéléromètre (6), et laissant ainsi les actionneurs (3) dans la position qu'ils avaient avant le passage à l'état de veille.

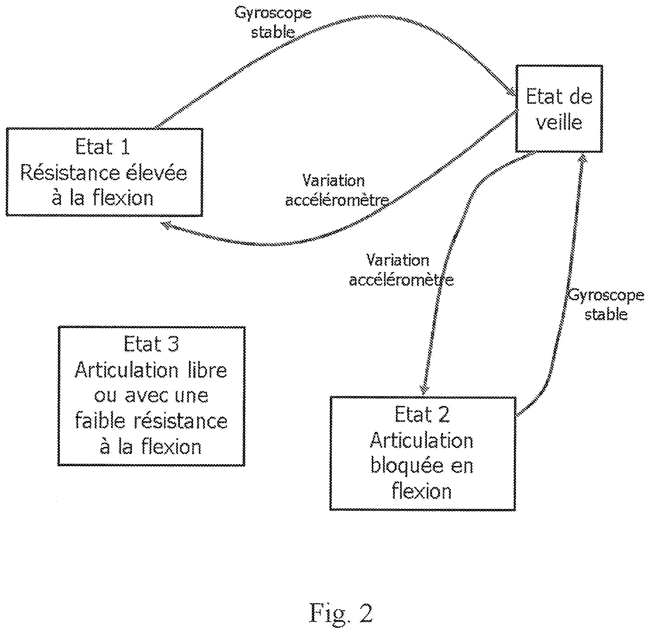

En effet, tel qu'illustré à la figure 2, pendant une période de temps déterminée, le microcontrôleur (4) surveille la mesure donnée par le gyroscope (5) de sorte que ledit microcontrôleur (4) passe à l'état de veille lorsqu'il détecte que le gyroscope (5) est stable, c'est-à-dire que la valeur absolue de la mesure donnée par le gyroscope (5) est inférieure à un seuil donné pendant la période de temps déterminée.

La sortie de l'état de veille s'effectue par l'intermédiaire de l'accéléromètre (6) qui détecte une variation de l'accélération mesurée par l'accéléromètre (6) du, soit à un choc sur le tibia, soit à un changement de l'angle du tibia par rapport à la verticale.

Avantageusement, et en référence à la figure 2, lorsque le microcontrôleur (4) détecte d'une part, un état permettant d'assurer la sécurité de l'utilisateur pendant l'état de veille, tel qu'un état dans lequel la résistance à la flexion de genou est à une valeur élevée permettant de freiner ou de bloquer le genou sous charge et, d'autre part, que le gyroscope (5) est stable, c'est à dire que la valeur absolue de la mesure donnée par le gyroscope (5) est inférieure à un seuil donné pendant la période de temps déterminée, ledit microcontrôleur (4) passe à l'état de veille afin d'économiser de l'énergie et d'augmenter l'autonomie de fonctionnement de ladite prothèse (1) en laissant la résistance à la flexion du genou à une valeur élevée. Ainsi si l'utilisateur change brutalement de position ou trébuche, la flexion du genou est contrôlée par une résistance élevée avant même que le microcontrôleur (4) sorte de l'état de veille.

Lors de la marche sur sol plat, en pente ou dans les escaliers, les états qui présentent une résistance élevée au mouvement d'une articulation (2) correspondent généralement à la phase d'appui, à l'exception de la fin de cette phase qui correspond notamment à la phase d'initiation de la phase pendulaire.

Une autre situation pour une personne appareillée correspond à une phase en appui dans laquelle le genou n'est pas en extension et la personne est en appui sur sa prothèse (1). Dans cette situation, si une partie de sa prothèse (1) est maintenue immobile pendant la période de temps déterminée, cela signifie que l'utilisateur cherche volontairement à immobiliser son articulation (2). Il est alors intéressant d'augmenter la résistance à la flexion jusqu'à bloquer le mouvement de l'articulation (2) par exemple dans le sens de la flexion pour le genou, avant que le microcontrôleur (4) ne passe à l'état de veille.

L'accéléromètre (6) peut être le seul organe électronique à rester alimenté dans le but de permettre le retour à l'état de fonctionnement normal du microcontrôleur (4). Pendant la période de temps déterminée durant laquelle le microcontrôleur (4) surveille le gyroscope (5), en parallèle l'accéléromètre (6) calcule et enregistre la moyenne de l'accélération sur cette même période de temps, de sorte que lorsque l'accéléromètre (6) détecte un écart déterminé entre la valeur de l'accélération mesurée pendant l'état de veille et la valeur de la moyenne de l'accélération enregistrée avant le passage à l'état de veille du microcontrôleur (4), l'accéléromètre (6) génère un signal apte à faire passer le microcontrôleur (4) à l'état de fonctionnement normal, dans des conditions correspondant à l'état dans lequel était le microcontrôleur (4) avant de passer à l'état de veille, en rétablissant l'alimentation électrique normale du ou des organes électroniques de la prothèse (1). L'accéléromètre (6) possède une faible consommation d'énergie par rapport aux autres organes électroniques. Par exemple l'accéléromètre (6) utilisé peut être celui commercialisé sous la référence « LIS3DH » par la société STMicroelectronics, qui intègre une logique de contrôle permettant la réalisation des étapes décrites ci-dessus. Sa consommation typique est de 11µA.

De cette manière, l'accéléromètre (6) est capable de calculer et d'enregistrer lui-même les informations relatives aux moyennes de l'accélération car il embarque un calculateur. Le calculateur embarqué dans l'accéléromètre (6) est capable de faire, de manière autonome, le calcul des moyennes et de la variation entre les moyennes. L'accéléromètre (6) consomme très peu d'énergie, même avec le calculateur spécifique intégré. Pendant l'état de veille, le calculateur principal de la prothèse (1) est également coupé car l'accéléromètre (6) inclut des moyens suffisants de calcul. Ainsi, la sortie de l'état de veille se fait par la détection d'une variation de la moyenne de l'accélération mesurée par rapport à la moyenne de l'accélération avant l'entrée en veille. De cette manière, la prothèse (1) ne se réactive pas de manière inopinée lorsqu'elle reçoit un léger choc. La sortie de l'état de veille est calculée de manière relative par rapport à l'environnement et la situation de la prothèse (1) avant la veille.

Le procédé selon l'invention permet donc de commander la mise en veille ou la sortie de la veille par l'utilisation conjointe d'un gyroscope (5) et d'un accéléromètre (6). Le gyroscope (5) permet la mise en veille en évaluant l'absence de mouvement de la prothèse (1) et l'accéléromètre (6) permet la sortie de la veille en évaluant la variation d'accélération par rapport à celle au moment de la mise en veille.

Bien entendu, pour un bon fonctionnement du procédé selon l'invention et de la machine d'états sur laquelle se base le logiciel embarqué dans le microcontrôleur (4), il convient de choisir des composants électroniques pour la prothèse (1) ayant un temps de réveil relativement faible pour ne pas compromettre la sécurité de l'utilisateur.

Enfin, il est bien évident que les exemples qui viennent d'être donnés ne sont que des illustrations particulières en aucun cas limitatives quant aux domaines d'application de l'invention. L'invention peut bien entendu être adaptée à d'autres domaines, telle que la robotique par exemple.

## Revendications

1. Procédé de commande d'une prothèse (1), telle qu'une prothèse de genou, de cheville ou de genou-cheville, comprenant au moins une articulation (2) contrôlée par un actionneur (3) piloté par un microcontrôleur (4) à partir de données issues d'au moins un gyroscope (5) et un accéléromètre (6) aptes à mesurer respectivement la vitesse angulaire et l'accélération d'au moins une partie de la prothèse (1), ***caractérisé* en ce que**, pendant une période de temps déterminée, le microcontrôleur (4) surveille la mesure donnée par le gyroscope (5) de sorte que ledit microcontrôleur (4) passe dans un état de veille en réduisant ou en coupant l'alimentation électrique d'au moins un organe électronique de la prothèse (1), à l'exception de celle de l'accéléromètre (6), lorsque ledit microcontrôleur (4) détecte que la valeur absolue de la mesure donnée par le gyroscope (5) est inférieure à un seuil donné pendant la période de temps déterminée.

2. Procédé de commande selon la revendication 1, ***caractérisé* en ce que** lorsque le microcontrôleur (4) passe à l'état de veille celui-ci réduit ou coupe l'alimentation électrique de tous les organes électroniques de la prothèse (1), à l'exception de l'accéléromètre (6).

3. Procédé de commande selon l'une quelconque des revendications 1 à 2, ***caractérisé* en ce que** le gyroscope (5) est agencé de sorte à mesurer la vitesse angulaire d'un segment lié à l'articulation (2) prothétique par rapport à un axe médio-latéral, l'accéléromètre (6) est agencé de sorte à mesurer l'accélération d'un segment lié à l'articulation (2) prothétique selon un axe antéro-postérieur, et le microcontrôleur (4) passe à l'état de veille lorsque la prothèse (1) est dans un état dans lequel la résistance de l'articulation (2) est supérieure à un seuil déterminé, ou dans un état dans lequel l'articulation (2) est bloquée.

4. Procédé de commande selon l'une quelconque des revendications 1 à 3, ***caractérisé* en ce que**, pendant la période de temps déterminée, l'accéléromètre (6) calcule et enregistre la moyenne de l'accélération, de sorte que lorsque l'accéléromètre (6) détecte un écart déterminé entre la valeur de l'accélération mesurée pendant l'état de veille et la valeur de la moyenne de l'accélération enregistrée avant le passage à l'état de veille du microcontrôleur (4), l'accéléromètre (6) génère un signal apte à faire passer le microcontrôleur (4) à nouveau dans un état de fonctionnement normal en rétablissant l'alimentation électrique normale du ou des organes électroniques de la prothèse (1).

5. Procédé de commande selon la revendication 4, ***caractérisé* en ce que** le signal généré par l'accéléromètre (6) permet au microcontrôleur (4) de passer à nouveau à l'état de fonctionnement normal et dans des conditions correspondant à l'état dans lequel était le microcontrôleur (4) avant de passer à l'état de veille.

6. Prothèse (1), telle qu'une prothèse de genou, de cheville ou de genou-cheville, comprenant au moins une articulation (2) contrôlée par un actionneur (3) piloté par un microcontrôleur (4) à partir de données issues d'au moins un gyroscope (5) et un accéléromètre (6) aptes à mesurer respectivement la vitesse angulaire et l'accélération d'au moins une partie de la prothèse (1), ***caractérisée* en ce qu'**elle comprend un microcontrôleur (4) apte à surveiller, pendant une période de temps déterminée, la mesure donnée par le gyroscope (5) et à passer dans un état de veille en réduisant ou en coupant l'alimentation électrique d'au moins un organe électronique de la prothèse (1), à l'exception de celle de l'accéléromètre (6), lorsque ledit microcontrôleur (4) détecte que la valeur absolue de la mesure donnée par le gyroscope (5) est inférieure à un seuil donné pendant la période de temps déterminée.

## Patentansprüche

1. Verfahren zum Steuern einer Prothese (1), wie beispielsweise einer Knie-, Knöchel- oder Knöchel-/Knieprothese, umfassend mindestens ein Gelenk (2), das von einem Stellglied (3) gesteuert wird, welches von einem Mikrocontroller (4) gesteuert wird, der auf Daten von mindestens einem Gyroskop (5) und einen Beschleunigungssensor (6) basiert, die geeignet sind, die Winkelgeschwindigkeit und Beschleunigung von mindestens einem Teil der Prothese (1) zu messen, **dadurch gekennzeichnet, dass** der Mikrocontroller (4) während eines bestimmten Zeitraums die vom Gyroskop (5) übermittelte Messung überwacht, und dass der Mikrocontroller (4) in einen Standby-Zustand wechselt, indem er die Stromversorgung mindestens einer elektronischen Komponente der Prothese (1), mit Ausnahme derjenigen des Beschleunigungssensors (6), reduziert oder abschaltet, wenn der Mikrocontroller (4) erkennt, dass der Absolutwert der vom Gyroskop (5) gegebenen Messung während des bestimmten Zeitraums unter einem bestimmten Schwellenwert liegt.

2. Steuerverfahren nach Anspruch 1, **dadurch gekennzeichnet, dass** der Mikrocontroller (4) im Standby-Modus die Stromversorgung aller elektronischen Komponenten der Prothese (1) mit Ausnahme des Beschleunigungssensors (6) reduziert oder unterbricht.

3. Steuerverfahren nach einem der Ansprüche 1 bis 2, **dadurch gekennzeichnet, dass** das Gyroskop (5) angeordnet ist, um die Winkelgeschwindigkeit eines an das Prothesengelenk (2) gebundenen Segments in Bezug auf eine medio-laterale Achse zu messen, der Beschleunigungssensor (6) angeordnet ist, um die Beschleunigung eines Segments, das sich auf das Prothesengelenk (2) bezieht, entlang einer posterior-anterioren Achse zu messen, und der Mikrocontroller (4) in den Standby-Zustand wechselt, wenn sich die Prothese (1) in einem Zustand, in dem der Widerstand des Gelenks (2) größer als ein bestimmter Schwellenwert ist, oder in einem Zustand, in dem das Gelenk (2) blockiert ist, befindet.

4. Steuerverfahren nach einem der Ansprüche 1 bis 3, **dadurch gekennzeichnet, dass** der Beschleunigungssensor (6) während des bestimmten Zeitraums die durchschnittliche Beschleunigung berechnet und aufzeichnet, so dass, wenn der Beschleunigungssensor (6) eine bestimmte Abweichung zwischen dem Wert der im Standby-Zustand gemessenen Beschleunigung und dem Wert der durchschnittlichen Beschleunigung, der vor dem Eintritt des Mikrocontrollers (4) in den Standby-Zustand aufgezeichnet wurde, erfasst, der Beschleunigungssensor (6) ein Signal erzeugt, das in der Lage ist, den Mikrocontroller (4) in einen normalen Betriebszustand zurückzusetzen, indem die normale Stromversorgung der elektronischen Komponente(n) der Prothese (1) wiederhergestellt wird.

5. Steuerverfahren nach Anspruch 4, **dadurch gekennzeichnet, dass** das vom Beschleunigungssensor (6) erzeugte Signal es dem Mikrocontroller (4) ermöglicht, in den normalen Betriebszustand zurückzukehren, und zwar unter Bedingungen, die dem Zustand entsprechen, in dem sich der Mikrocontroller (4) vor dem Eintritt in den Standby-Zustand befand.

6. Prothese (1), wie beispielsweise eine Knie-, Knöchel- oder Knie-Knöchelprothese, umfassend mindestens ein Gelenk (2), das von einem Stellglied (3) gesteuert wird, welches von einem Mikrocontroller (4) gesteuert wird, der auf Daten von mindestens einem Gyroskop (5) und einen Beschleunigungssensor (6) basiert, die geeignet sind, die Winkelgeschwindigkeit und Beschleunigung von mindestens einem Teil der Prothese (1) zu messen, **dadurch gekennzeichnet, dass** sie einen Mikrocontroller (4) umfasst, der geeignet ist, während eines bestimmten Zeitraums die vom Gyroskop (5) übermittelte Messung zu überwachen und in einen Standby-Zustand zu wechseln, indem er die Stromversorgung mindestens einer elektronischen Komponente der Prothese (1), mit Ausnahme derjenigen des Beschleunigungssensors (6), reduziert oder abschaltet, wenn der Mikrocontroller (4) erkennt, dass der Absolutwert der vom Gyroskop (5) gegebenen Messung während des bestimmten Zeitraums unter einem bestimmten Schwellenwert liegt.

## Claims

1. Method for controlling a prosthesis (1), such as a knee, ankle or knee/ankle prosthesis, comprising at least one joint (2) controlled by an actuator (3) controlled by a microcontroller (4) on the basis of data issuing from at least one gyroscope (5) and an accelerometer (6) that are able to measure the angular speed and the acceleration, respectively, of at least part of the prosthesis (1), ***characterized* in that**, during a defined period of time, the microcontroller (4) monitors the measurement given by the gyroscope (5) such that said microcontroller (4) enters an idle state reducing or cutting the electricity supply of at least one electronic component of the prosthesis (1), except that of the accelerometer (6), when said microcontroller (4) detects that the absolute value of the measurement given by the gyroscope (5) is below a given threshold during the defined period of time.

2. Method for controlling according to claim 1, ***characterized* in that** when the microcontroller (4) enters the idle state, it reduces or cuts the electricity supply of all of the electronic components of the prosthesis (1), with the exception of the accelerometer (6).

3. Method for controlling according to any one of claims 1 to 2, ***characterized* in that** the gyroscope (5) is arranged so as to measure the angular speed of a segment linked to the prosthetic joint (2) relative to a mediolateral axis, the accelerometer (6) is arranged so as to measure the acceleration of a segment linked to the prosthetic joint (2) along an anteroposterior axis, and the microcontroller (4) enters the idle state when the prosthesis (1) is in a state in which the resistance of the joint (2) is above a predefined threshold, or in a state in which the joint (2) is blocked.

4. Method for controlling according to any one of claims 1 to 3, ***characterized* in that**, during the defined period of time, the accelerometer (6) calculates and records the average acceleration, such that when the accelerometer (6) detects a defined deviation between the value of the acceleration measured during the idle state and the value of the average acceleration recorded before entering the idle state of the microcontroller (4), the accelerometer (6) generates a signal able to cause the microcontroller (4) to reenter a normal operating state by reestablishing the normal electricity supply to the electronic component (s) of the prosthesis (1).

5. Method for controlling according to claim 4, ***characterized* in that** the signal generated by the accelerometer (6) allows the microcontroller (4) to reenter the normal operating state and under conditions corresponding to the state that the microcontroller (4) was in before entering the idle state.

6. Prosthesis (1), such as a knee, ankle or knee/ankle prosthesis, comprising at least one joint (2) controlled by an actuator (3) controlled by a microcontroller (4) on the basis of data issuing from at least one gyroscope (5) and an accelerometer (6) that are able to measure the angular speed and the acceleration, respectively, of at least part of the prosthesis (1), ***characterized* in that** it comprises a microcontroller (4) able to monitor, during a defined period of time, the measurement given by the gyroscope (5) and to enter an idle state by reducing or cutting the electricity supply of at least one electronic component of the prosthesis (1), except that of the accelerometer (6), when said microcontroller (4) detects that the absolute value of the measurement given by the gyroscope (5) is below a given threshold during the defined period of time.
